# EUROPÄISCHE PATENTSCHRIFT

(11) **EP 1 529 041 B1**
(45) Veröffentlichungstag und Bekanntmachung des Hinweises auf die Patenterteilung: **11.10.2006**
(21) Anmeldenummer: 03784088.1
(22) Anmeldetag: 28.07.2003
(51) Int. Cl.: C07D 401/12, A61K 31/245, A61P 7/02

(54) **NEUE PRODRUGS VON 1-METHYL-2-(4-AMIDINOPHENYLAMINOMETHYL)-BENZIMIDAZOL-5-YL-CARBONSÄURE-(N -2-PYRIDIL-N-2-HYDROXYCARBONYLETHYL)-AMID, IHRE HERSTELLUNG UND IHRE VERWENDUNG ALS ARZNEIMITTEL**
NOVEL PRODRUGS OF 1-METHYL-2-(4-AMIDINOPHENYLAMINOMETHYL)-BENZIMIDAZOL-5-YL-CARBOXYLIC ACID-(N-2-PYRIDIL-N-2-HYDROXYCARBONYLETHYL)-AMIDE, PRODUCTION AND USE THEREOF AS MEDICAMENTS
NOUVEAUX PROMEDICAMENTS DE 1-METHYL-2-(4-AMIDINOPHENYLAMINOMETHYL)-BENZIMIDAZOL-5-YL-ACIDE CARBOXYLIQUE-(N-2-PYRIDIL-N-2-HYDROXYCARBONYLETHYL)-AMIDE, LEUR PREPARATION ET LEUR UTILISATION EN TANT QUE MEDICAMENTS

(30) Priorität: 02.08.2002 DE 10235639
(43) Veröffentlichungstag der Anmeldung: 11.05.2005
(73) Patentinhaber: Boehringer Ingelheim Pharma GmbH & Co. KG, 55216 Ingelheim am Rhein (DE)
(72) Erfinder: HAUEL, Norbert, 88433 Schemmerhofen (DE); BUSCH, Ulrich, 88400 Biberach (DE); COLBATZKY, Florian, 88441 Stafflangen (DE)
(86) Internationale Anmeldenummer: PCT/EP2003/008289
(87) Internationale Veröffentlichungsnummer: WO 2004/014894

(56) Entgegenhaltungen:
- WO-A-98/37075

## Beschreibung

Gegenstand der vorliegenden Erfindung sind neue Verbindungen der allgemeinen Formel 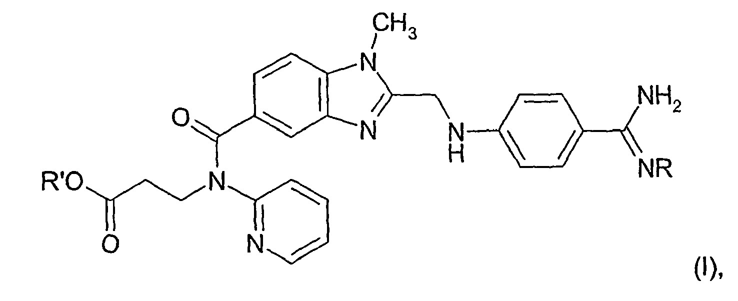 deren Tautomere und deren Salze, insbesondere deren physiologisch verträgliche Salze mit anorganischen oder organischen Säuren oder Basen, welche wertvolle Eigenschaften aufweisen.

Bei den Verbindungen der allgemeinen Formel I handelt es sich um Prodrugs der thrombinhemmenden Verbindung 1-Methyl-2-(4-amidinophenylaminomethyl)-benzimidazol-5-yl-carbonsäure-(*N*-2-pyridyl-*N*-2-hydroxycarbonylethyl)-amid (II), 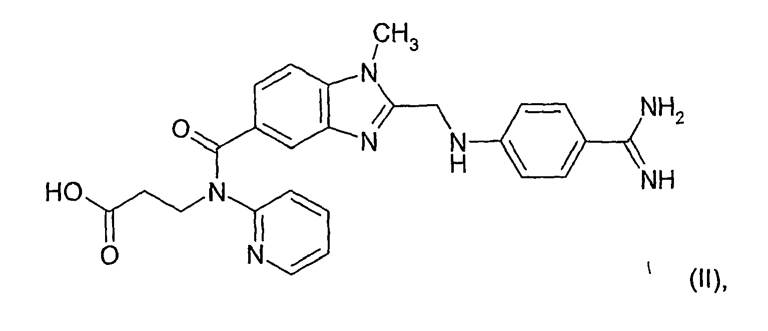 welche bereits aus der WO 98/37075 bekannt ist. Die erfindungsgemäßen Prodrugs sind insbesondere zur subkutanen Anwendung gut geeignet, da sie nach subkutaner Injektion gut verträglich sind, insbesondere bei subkutaner Anwendung keine lokalen Unverträglichkeiten an der Injektionsstelle hervorrufen.

Gegenstand der vorliegenden Anmeldung sind somit die neuen Verbindungen der obigen allgemeinen Formel I, deren Tautomere und deren Salze sowie deren Herstellung, die die pharmakologisch wirksamen Verbindungen enthaltenden Arzneimittel und deren Verwendung.

In der obigen allgemeinen Formel I bedeutet
a) R' ein Wasserstoffatom und R eine Methoxycarbonylgruppe
   oder
b) R' ein Wasserstoffatom oder eine C₁₋₆-Alkylgruppe und R eine Hydroxygruppe.

Die bei der vorstehenden Definition erwähnten Alkylgruppen, die mehr als 2 Kohlenstoffatome enthalten, schließen auch deren verzweigte Isomere wie beispielsweise die Isopropyl-, tert.Butyl- und Isobutylgruppe ein.

Bevorzugt sind diejenigen Verbindungen der allgemeinen Formel I, in denen
a) R' ein Wasserstoffatom und R eine Methoxycarbonylgruppe
   oder
b) R' ein Wasserstoffatom oder eine C₁₋₃ -Alkylgruppe und R eine Hydroxygruppe bedeuten, deren Tautomere und deren Salze.

Besonders bevorzugt ist die Verbindung 1-Methyl-2-[4-(*N*-hydroxyamidino)-phenyl-aminomethyl]-benzimidazol-5-yl-carbonsäure-(*N*-2-pyridyl-*N*-2-ethoxycarbonylethyl)-amid, deren Tautomere und deren Salze, insbesondere deren physiologisch verträglichen Salze.

Besonders bevorzugte Salze sind das Maleat, das Hydrochlorid und das Methansulfonat der Verbindung 1-Methyl-2-[4-(*N*-hydroxyamidino)-phenylaminomethyl]-benzimidazol-5-yl-carbonsäure-(*N*-2-pyridyl-*N*-2-ethoxycarbonylethyl)-amid.

Eine weitere besonders bevorzugte Verbindung ist 1-Methyl-2-[4-(*N-*methoxycarbonylamidino)-phenylaminomethyl]-benzimidazol-5-yl-carbonsäure-(*N*-2-pyridyl-*N*-2-hydroxycarbonylethyl)-amid, deren Tautomere und deren Salze, insbesondere deren physiologisch verträglichen Salze.

Besonders bevorzugte Salze sind das Maleat, das Methansulfonat und das Natriumsalz der Verbindung 1-Methyl-2-[4-(*N*-methoxycarbonylamidino)-phenylaminomethyl]-benzimidazol-5-yl-carbonsäure-(*N*-2-pyridyl-*N*-2-hydroxycarbonylethyl)-amid.

Die neuen Verbindungen lassen sich nach an sich bekannten Verfahren herstellen, beispielsweise nach folgenden Verfahren:

### a. Zur Herstellung einer Verbindung der allgemeinen Formel I, in der R eine Hydroxygruppe darstellt:

Umsetzung einer gegebenenfalls im Reaktionsgemisch gebildeten Verbindung der allgemeinen Formel 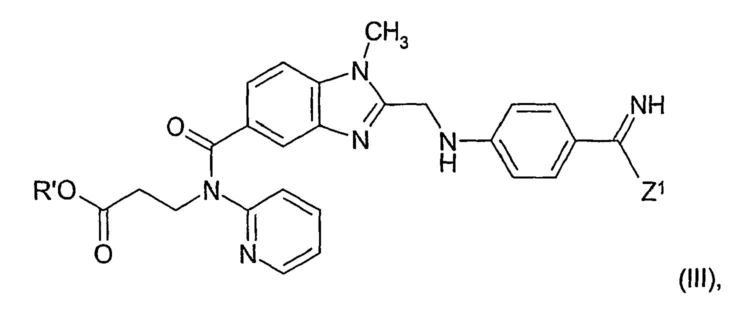 in der
R' wie eingangs definiert ist und
Z₁ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt, mit einem Amin der allgemeinen Formel

H₂N - OH (IV).

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einer Verbindung der allgemeinen Formel III oder mit einem entsprechenden Säureadditionssalz wie beispielsweise Ammoniumcarbonat durchgeführt.

Verbindungen der allgemeinen Formel III und ihre Herstellung sind beispielsweise in der WO 98/37075 beschrieben.

### b. Zur Herstellung einer Verbindung der allgemeinen Formel I, in der R' Wasserstoff bedeutet:

Überführung einer Verbindung der allgemeinen Formel 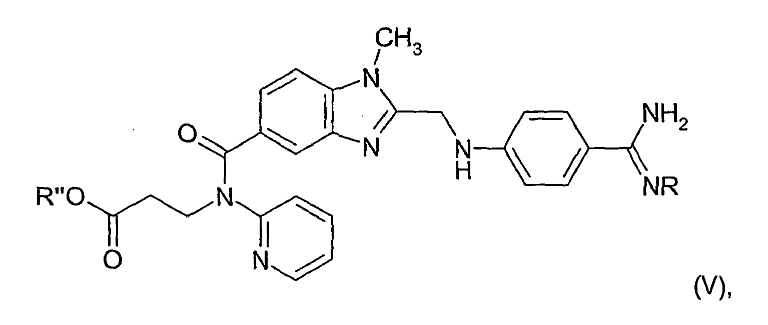 in der
R wie eingangs definiert ist und
R" eine durch Hydrolyse, Behandeln mit einer Säure oder Base, Thermolyse oder Hydrogenolyse in eine Carboxylgruppe überführbare Gruppe bedeutet,
mittels Hydrolyse, Behandeln mit einer Säure oder Base, Thermolyse oder Hydrogenolyse in eine Verbindung der allgemeinen Formel I, in der R' Wasserstoff bedeutet.

Als eine in eine Carboxygruppe überführbare Gruppe kommt beispielsweise eine durch einen Schutzrest geschützte Carboxylgruppe wie deren funktionelle Derivate, z. B. deren unsubstituierte oder substituierte Amide, Ester, Thioester, Trimethylsilylester, Orthoester oder Iminoester, welche zweckmäßigerweise mittels Hydrolyse in eine Carboxylgruppe übergeführt werden,
deren Ester mit tertiären Alkoholen, z.B. der tert.-Butylester, welche zweckmäßigerweise mittels Behandlung mit einer Säure oder Thermolyse in eine Carboxylgruppe übergeführt werden, und
deren Ester mit Aralkanolen, z.B. der Benzylester, welche zweckmäßigerweise mittels Hydrogenolyse in eine Carboxylgruppe übergeführt werden, in Betracht.

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Enthält R" in einer Verbindung der Formel V beispielsweise die tert.-Butyl- oder tert.-Butyloxycarbonylygruppe, so können diese auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden.

Enthält R" in einer Verbindung der Formel V beispielsweise die Benzyl- oder Benzyloxycarbonylgruppe, so können diese auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden.

Verbindungen der allgemeinen Formel V und ihre Herstellung sind beispielsweise in der WO 98/37075 beschrieben.

### c. Zur Herstellung einer Verbindung der allgemeinen Formel I, in der R eine Methoxycarbonylgruppe bedeutet:

Umsetzung einer Verbindung der allgemeinen Formel 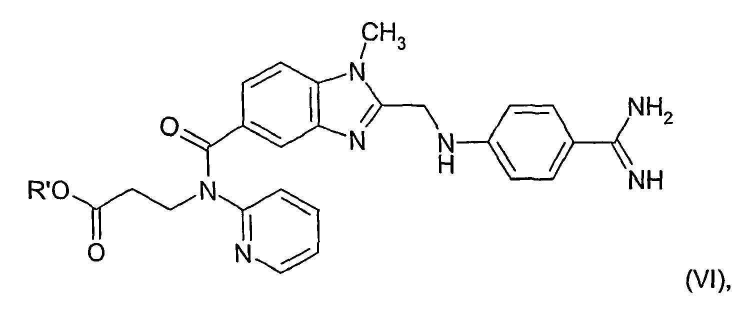 in der
R' wie eingangs definiert ist, mit einer Verbindung der allgemeinen Formel

Z₂-COOCH₃ , (VII)

in der
Z₂ eine nukleofuge Austrittsgruppe wie ein Halogenatom, z.B. ein Chlor-, Brom- oder Jodatom, bedeutet.

Die Umsetzung wird vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch vozugsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/-Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Die als Ausgangsstoffe verwendeten Verbindungen der allgemeinen Formeln III bis VII, welche literaturbekannt sind, erhält man nach literaturbekannten Verfahren, des weiteren wird ihre Herstellung in der WO 98/37075 beschrieben.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure, Methansulfonsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die neuen Verbindungen der allgemeinen Formel I und deren Salze als Prodrugs der Wirksubstanz II wertvolle Eigenschaften auf, da sie nach oraler oder parenteraler Gabe in die thrombinhemmende Wirksubstanz II umgewandelt werden. Insbesondere zeichnen sie sich durch eine gute Verträglichkeit nach subkutaner Applikation aus.

Beispielsweise wurden die Verbindungen
A = 1-Methyl-2-[4-(*N*-hydroxyamidino)-phenylaminomethyl]-benzimidazol-5-yl-carbonsäure-(*N*-2-pyridyl-*N*-2-ethoxycarbonylethyl)-amid und
B = 1-Methyl-2-[4-(*N*-methoxycarbonylamidino)-phenylaminomethyl]-benzimidazol-5-yl-carbonsäure-(*N*-2-pyridyl-*N*-2-hydroxycarbonylethyl)-amid
im Vergleich zur Wirksubstanz II auf ihre Verträglichkeit nach subkutaner Anwendung wie folgt untersucht:

Lösungen der zu testenden Substanzen wurden je zwei weiblichen Kaninchen (Alter: 11 bis 14 Wochen, Gewicht: 2.0 bis 3.5 kg) je einmal an drei aufeinanderfolgenden Tagen subkutan appliziert. Gleichzeitig mit den Applikationen wurde kontralateral jeweils eine Plazebolösung am gleichen Tier injiziert. Die Tiere wurden 48 Stunden nach der letzten Applikation getötet und seziert. Die Injektionsstellen der Substanz-Applikationen wurden histologisch mit denen der Plazebogaben verglichen.

Im Gegensatz zu Verbindung II, die an den Injektionsstellen deutliche Entzündungserscheinungen hervorrief, erwiesen sich die Verbindungen A und B als sehr gut lokal verträglich.

Zur Untersuchung der Umwandlung der Prodrugs A und B in die Wirksubstanz II wurden den Versuchstieren zu verschiedenen Zeitpunkten nach der subkutanen Applikation mit K-EDTA-Spritzen jeweils 1.0 ml Blut aus der zentralen Ohrarterie entnommen. Das Blut wurde zentrifugiert und das Plasma mit dem gleichen Volumen 0.2 M Salzsäure angesäuert. Die Lagerung dieser Lösungen erfolgte bei -20°C. Die Messung der Konzentrationen der Wirksubstanz II erfolgte mit Hilfe einer HPLC-MS/MS-Apparatur (Perkin Elmer Sciex API 300 LC-MS/MS system). Die Nachweisgrenze lag bei 4 ng/ml. Auf diese Weise wurde festgestellt, daß die Prodrugs A und B nach subkutaner Injektion in den Versuchstieren in die Wirksubstanz II umgewandelt wurden.

Aufgrund ihrer pharmakologischen Eigenschaften eignen sich die neuen Verbindungen und deren physiologisch verträglichen Salze zur Vorbeugung und Behandlung venöser und arterieller thrombotischer Erkrankungen, wie zum Beispiel der Behandlung von tiefen Beinvenen-Thrombosen, der Verhinderung von Reocclusionen nach Bypass-Operationen oder Angioplastie (PT(C)A), sowie der Occlusion bei peripheren arteriellen Erkrankungen wie Lungenembolie, der disseminierten intravaskulären Gerinnung, der Prophylaxe der Koronarthrombose, der Prophylaxe des Schlaganfalls und der Verhinderung der Occlusion von Shunts oder Stents. Zusätzlich sind die erfindungsgemäßen Verbindungen zur antithrombotischen Unterstützung bei einer thrombolytischen Behandlung, wie zum Beispiel mit rt-PA oder Streptokinase, zur Verhinderung der Langzeitrestenose nach PT(C)A, zur Verhinderung der Metastasierung und des Wachstums von koagulationsabhängigen Tumoren und von fibrinabhängigen Entzündungsprozessen wie zum Beispiel Arthritis geeignet.

Die zur Erzielung einer entsprechenden Wirkung erforderliche Dosierung beträgt zweckmäßigerweise bei subkutaner Gabe 0.03 bis 10.0 mg/kg, vorzugsweise 0.05 bis 3.0 mg/kg, bei intravenöser Gabe 0.1 bis 3.0 mg/kg, vorzugsweise 0.3 bis 1.0 mg/kg und bei oraler Gabe 0.1 bis 50.0 mg/kg, vorzugsweise 0.3 bis 10.0 mg/kg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Wasser/Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Zäpfchen oder injizierbare Lösungen einarbeiten. Bevorzugt ist die Einarbeitung in Verdünnungsmittel wie beispielsweise Wasser, Wasser/Ethanol, Wasser/Glycerin, Wasser/Sorbit, Wasser/Polyethylenglykol oder Wasser/Propylenglykol zur Herstellung einer injizierbaren Lösung.

Die nachfolgenden Beispiele sollen die Erfindung näher erläutern:

### Beispiel 1

### 1-Methyl-2-[4-(N-methoxycarbonylamidino)-phenylaminomethyl]-benzimidazol-5-yl-carbonsäure-(N-2-pyridyl-N-2-hydroxycarbonylethyl)-amid

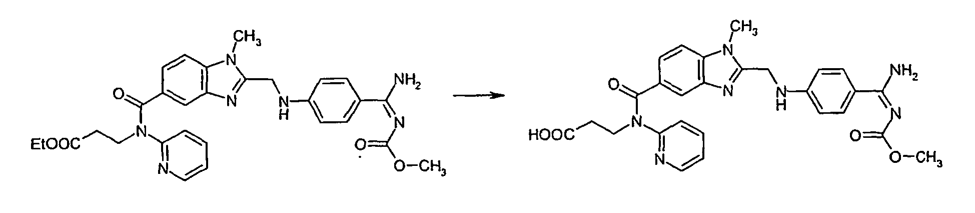

Zu einer Suspension von 8.50 g (15.24 mmol) 1-Methyl-2-[4-(N-methoxycarbonyl-amidino)-phenylaminomethyl]-benzimidazol-5-yl-carbonsäure-(*N*-2-pyridyl-*N*-2-ethoxycarbonylethyl)-amid (zur Herstellung siehe WO 98/37075) in 140 ml Tetrahydrofuran wurde unter Rühren bei Raumtemperatur eine Lösung von 0.71 g (17.0 mMol) Lithiumhydroxid-Hydrat in 175 ml Wasser gegeben. Die so entstandene klare Lösung wurde weitere zwei Stunden bei Raumtemperatur gerührt, anschließend am Rotationsverdampfer ca. ein Drittel des Volumens abgedampft, die verbleibende Lösung mit ca 200 ml Wasser verdünnt und mit Salzsäure auf pH 5 bis 6 eingestellt. Der ausgefallene Feststoff wurde abgesaugt, in einem Gemisch aus Methanol und Dichlormethan (1 : 1) gelöst, ungelöste Bestandteile abfiltriert und das Filtrat zur Trockne eingedampft. Das so erhaltene Produkt wurde mit Aceton verrieben, abgesaugt, mit Aceton und Diethylether gewaschen und getrocknet.
Ausbeute: 80.5% der Theorie.
C₂₇H₂₇N₇O₅ (529.56)

| | |
|---|---|
| Massenspektrum: | (M+H)⁺ = 530 |
| | (M-H)⁻ = 528 |
| | (M+Na)⁺ = 552 |

¹H-NMR (d₆-DMSO): δ = 2.61 (t, 2H); 3.58 (s, 3H); 3.77 (s,3H); 4.19 (t, 2H); 4.60 (d, 2H); 6.76 (d, 2H); 6.96 (m, 2H); 7.09 bis 7.10 (m, 2H); 7.40 (d, 1 H); 7.47 (s, 1 H); 7.56 (t, 1 H); 7.80 (d, 2H); 8.38 (m, 1 H); 8.50 bis 9.20 (breites d, 2H) ppm; Carboxyl-H nicht sichtbar.

### Beispiel 2

### 1-Methyl-2-[4-(N-methoxycarbonylamidino)-phenylaminomethyl]-benzimidazol-5-yl-carbonsäure-(N-2-pyridyl-N-2-hydroxycarbonylethyl)-amid - Methansulfonat

Zu einer Suspension von 1.00 g (1.89 mmol) 1-Methyl-2-[4-(*N*-methoxycarbonyl-amidino)-phenylaminomethyl]-benzimidazol-5-yl-carbonsäure-(*N*-2-pyridyl-N-2-hydroxycarbonylethyl)-amidin 35 ml Methanol wurde unter Rühren bei Raumtemperatur eine Lösung von 181 mg (1.89 mmol) Methansulfonsäure in 5 ml Methanol gegeben, wobei eine klare Lösung entstand. Nach einer Stunde wurde die Lösung filtriert, Das Filtrat auf ca. 10 ml eingeengt und danach Aceton zugetropft, bis eine leichte Trübung einsetzte. Nach einer weiteren Stunde Rühren bei Raumtemperatur wurde das ausgefallene Produkt abgesaugt, mit Aceton und Diethylether gewaschen und getrocknet.
Ausbeute: 84.7% der Theorie.
C₂₇H₂₇N₇O₅ x CH₄O₃S (625.67)

| | |
|---|---|
| Massenspektrum: | (M+H)⁺ = 530 |
| | (M+CH₃SO₃⁻)⁻ = 624 |

Schmelzpunkt: ab 214°C Zersetzung

### Beispiel 3

### 1-Methyl-2-[4-(N-methoxycarbonylamidino)-phenylaminomethyl]-benzimidazol-5-yl-carbonsäure-(N-2-pyridyl-N-2-hydroxycarbonylethyl)-amid - Maleat

Analog zu Beispiel 2 hergestellt aus 1-Methyl-2-[4-(*N*-methoxycarbonylamidino)-phenylaminomethyl]-benzimidazol-5-yl-carbonsäure-(*N*-2-pyridyl-*N*-2-hydroxy-carbonylethyl)-amid und Maleinsäure.
Ausbeute: 69.2% der Theorie.
C₂₇H₂₇N₇O₅ x C₄H₄O₄ (645.63)

| | |
|---|---|
| Massenspektrum: | (M+H)⁺ = 530 |
| | (M-H)⁻ = 528 |
| | (M+C₄H₃O₄⁻)⁻ = 644 |

Schmelzpunkt: 179 -180°C

### Beispiel 4

### 1-Methyl-2-[4-(N-methoxycarbonylamidino)-phenylaminomethyl]-benzimidazol-5-yl-carbonsäure-(N-2-pyridyl-N-2-hydroxycarbonylethyl)-amid - Natriumsalz

Zu einer Suspension von 1.20 g (2.27 mmol) 1-Methyl-2-[4-(N-methoxycarbonyl-amidino)-phenylaminomethyl]-benzimidazol-5-yl-carbonsäure-(*N*-2-pyridyl-*N*-2-hydroxycarbonylethyl)-amid in 30 ml Ethanol wurde unter Rühren bei Raumtemperatur eine Lösung von 90.6 mg (2.27 mmol) Natriumhydroxid in 1.0 ml Wasser gegeben. Es entstand eine klare Lösung, aus der durch langsames Zutropfen von Diethylether das Produkt ausgefällt wurde. Nach dem Abfiltrieren wurde das Produkt mit ca. 20 ml Diethylether gewaschen und bei 60°C getrocknet.
Ausbeute: 72.3% der Theorie
Schmelzpunkt: amorph
C₂₇H₂₆N₇O₅Na x H₂O (569.54)

| | | | |
|---|---|---|---|
| Elementaranalyse: | | | |
| berechnet: | C 56.94 | H 4.96 | N 17.78 |
| gefunden: | C 56.68 | H 5.17 | N 17.55 |

¹H-NMR (d₆-DMSO): δ = 2.20 (t, 2H); 3.59 (s, 3H); 3.76 (s, 3H); 4.10 (t, 2H); 4.60 (d, 2H); 6.77 (d, 2H); 7.00 - 7.13 (m, 3H); 7.17 (d, 1 H); 7.38 (d, 1 H); 7.48 (s, 1 H); 7.57 (t, 1 H); 7.80 (d, 2H); 8.34 (m, 1 H); 8.60 - 9.22 (d, breit, 2H) ppm.

### Beispiel 5

### 1-Methyl-2-[4-(N-hydroxyamidino)-phenylaminomethyl]-benzimidazol-5-yl-carbonsäure-(N-2-pyridyl-N-2-ethoxycarbonylethyl)-amid

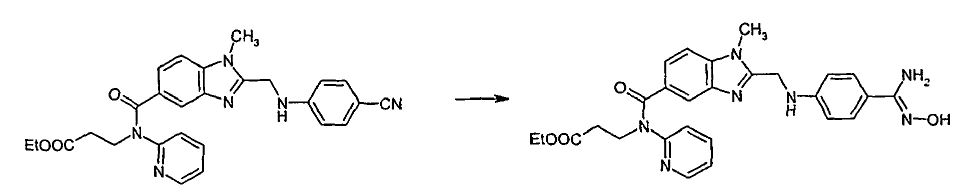

Zu einer unter Eiskühlung gesättigten Lösung von HCl-Gas in Ethanol (500 ml) wurden unter Rühren 20.0 g (41.45 mmol) 1-Methyl-2-(4-cyano-phenylaminomethyl)-benzimidazol-5-yl-carbonsäure-(*N*-2-pyridyl-*N*-2-ethoxycarbonylethyl)-amid (zur Herstellung siehe WO 98/37075) gegeben, das Kühlbad entfernt und weitere fünf Stunden gerührt. Das Reaktionsgemisch wurde dann am Rotationsverdampfer im Vakuum eingeengt, wobei die Temperatur stets unter 30°C gehalten wurde. Der Rückstand wurde in 200 ml Ehanol gelöst und unter Eiskühlung langsam mit 20.0 g (198 mMol) Triethylamin versetzt. Dann wurden 3.75 g (54.0 mMol) Hydroxylamin-Hydrochlorid zugegeben und zwei Stunden bei Raumtemperatur gerührt. Das dabei ausgefallene Produkt wurde abgesaugt und aus Ethanol /Dichlormethan (2 : 1) umkristallisiert.
Ausbeute: 61.5% der Theiorie
Schmelzpunkt: 162 - 164°C
C₂₇H₂₉N₇O₄ (515.58)

| | |
|---|---|
| Massenspektrum: | (M+H)⁺ = 516 |
| | (M+Na)⁺ = 538 |

¹H-NMR (d₆-DMSO): δ = 1.12 (t, 3H); 2.69 (t, 2H); 3.76 (s, 3H); 3.98 (q, 2H); 4.22 (t, 2H); 4.52 (d, 2H); 5.55 (s, breit, 2H); 6.43 (t, 1 H); 6.70 (d, 2H); 6.89 (d, 1 H); 7.09 bis 7.20 (m, 2H); 7.40 (m, 3H); 7.48 (s, 1 H); 7.55 (t, 1 H); 8.40 (m, 1 H); 9.24 (s, 1 H) ppm.

Analog zu Beispiel 5 können hergestellt werden:
1-Methyl-2-[4-(*N*-hydroxyamidino)-phenylaminomethyl]-benzimidazol-5-yl-carbonsäure-(*N*-2-pyridyl-*N*-2-methoxycarbonylethyl)-amid,
1-Methyl-2-[4-(*N*-hydroxyamidino)-phenylaminomethyl]-benzimidazol-5-yl-carbonsäure-(*N*-2-pyridyl-*N*-2-n-propyloxycarbonylethyl)-amid,
1-Methyl-2-[4-(*N*-hydroxyamidino)-phenylaminomethyl]-benzimidazol-5-yl-carbonsäure-(*N*-2-pyridyl-*N*-2-isopropyloxycarbonylethyl)-amid,
1-Methyl-2-[4-(*N*-hydroxyamidino)-phenylaminomethyl]-benzimidazol-5-yl-carbonsäure-(*N*-2-pyridyl-*N*-2-n-butyloxycarbonylethyl)-amid,
1-Methyl-2-[4-(*N*-hydroxyamidino)-phenylaminomethyl]-benzimidazol-5-yl-carbonsäure-(*N*-2-pyridyl-*N*-2-isobutyloxycarbonylethyl)-amid.

### Beispiel 6

### 1-Methyl-2-[4-(N-hydroxyamidino)-phenylaminomethyl]-benzimidazol-5-yl-carbonsäure-(N-2-pyridyl-N-2-hydroxycarbonylethyl)-amid

Hergestellt analog zu Beispiel 1 aus 1-Methyl-2-[4-(*N*-hydroxyamidino)-phenylamino-methyl]-benzimidazol-5-yl-carbonsäure-(*N*-2-pyridyl-*N*-2-ethoxycarbonylethyl)-amid. Ausbeute: 66.2% der Theorie.
C₂₅H₂₅N₇O₄ (487.58)

| | |
|---|---|
| Massenspektrum: | (M+H)⁺ = 488 |
| | (M-H)⁻ = 486 |
| | (M+Na)⁺ = 510 |

### Beispiel 7

### 1-Methyl-2-[4-(N-hydroxyamidino)-phenylaminomethyl]-benzimidazol-5-yl-carbonsäure-(N-2-pyridyl-N-2-ethoxycarbonylethyl)-amid - Hydrochlorid

Eine Lösung von HCl in Ethanol wurde erzeugt, indem 152 mg (1.94 mmol) Acetylchlorid in 5.0 ml Ethanol eingerührt wurden. Diese wurde bei Raumtemperatur zu einer Lösung von 1.0 g (1.94 mmol) 1-Methyl-2-[4-(*N*-hydroxyamidino)-phenylamino-methyl]-benzimidazol-5-yl-carbonsäure-(N-2-pyridyl-N-2-ethoxycarbonylethyl)-amid in 50 ml absolutem Ethanol hinzugefügt, anschließend am Rotationsverdampfer auf ca. 10 ml Volumen eingeengt und dann unter Rühren so lange Essigsäureethylester zugetropft, bis eine schwache Trübung zu beobachten war. Das Gemisch wurde ca. weitere 15 Stunden gerührt, dann das ausgefallene Produkt abgesaugt, mit Diethylether gewaschen und getrocknet.
Ausbeute: 78.3% der Theorie.
Schmelzpunkt: 155 - 157°C

| | |
|---|---|
| Massenspektrum: | (M+H)⁺ = 516 |
| | (M+Na)⁺ = 538 |

C₂₇H₂₉N₇O₄ x HCl x H₂O (570.05)

| Elementaranalyse: | | | | |
|---|---|---|---|---|
| berechnet: | C 56.89 | H 5.66 | N 17.20 | Cl 6.22 |
| gefunden: | C 56.80 | H 5.67 | N 17.06 | Cl 6.25 |

### Beispiel 8

### 1-Methyl-2-[4-(N-hydroxyamidino)-phenylaminomethyl]-benzimidazol-5-yl-carbonsäure-(N-2-pyridyl-N-2-ethoxycarbonylethyl)-amid - Maleat

In 100 ml absolutem Ethanol wurde 1.0 g (1.94 mmol) 1-Methyl-2-[4-(N-hydroxy-amidino)-phenylaminomethyl]-benzimidazol-5-yl-carbonsäure-(N-2-pyridyl-N-2-ethoxycarbonylethyl)-amid unter Erwärmung gelöst und 225 mg (1.94 mmol) Maleinsäure hinzugegeben. Die Lösung wurde dann auf ca. 15 ml Volumen eingeengt und bei Raumtemperatur so lange Essigsäureethylester zugetropft, bis eine schwache Trübung zu erkennen war. Nach Rühren über Nacht wurde das ausgefallene Produkt abgesaugt, mit Diethylether gewaschen und getrocknet. Ausbeute: 61.0% der Theorie.
Schmelzpunkt: amorph.
C₂₇H₂₉N₇O₄ x C₄H₄O₄

| | |
|---|---|
| Massenspektrum: | (M+H)⁺ = 516 |
| | (M+Na)⁺ = 538 |

### Beispiel 9

### 1-Methyl-2-[4-(N-hydroxyamidino)-phenylaminomethyl]-benzimidazol-5-yl-carbonsäure-(N-2-pyridyl-N-2-ethoxycarbonylethyl)-amid - Methansulfonat

Hergestellt analog Beispiel 8 aus 1-Methyl-2-[4-(*N*-hydroxyamidino)-phenylamino-methyl]-benzimidazol-5-yl-carbonsäure-(*N*-2-pyridyl-*N*-2-ethoxycarbonylethyl)-amid und Methansulfonsäure.
Ausbeute: 67.4% der Theorie.
Schmelzpunkt: 128 - 130°C.

| | |
|---|---|
| Massenspektrum: | (M+H)⁺ = 516 |
| | (M+Na)⁺ = 538 |

### Beispiel 10

### Trockenampulle mit 75 mq Wirkstoff pro 10 ml

### Zusammensetzung:

| | |
|---|---|
| Wirkstoff | 75,0 mg |
| Mannitol | 50,0 mg |
| Wasser für Injektionszwecke | ad 10,0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel 11

### Trockenampulle mit 35 mg Wirkstoff pro 2 ml

### Zusammensetzung:

| | |
|---|---|
| Wirkstoff | 35,0 mg |
| Mannitol | 100,0 mg |
| Wasser für Injektionszwecke | ad 2,0 ml |

### Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

### Beispiel 12

### Tablette mit 50 mg Wirkstoff

### Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

### Beispiel 13

### Tablette mit 350 mg Wirkstoff

### Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

### Beispiel 14

### Kapseln mit 50 mg Wirkstoff

### Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2.0 mg |
| | 160,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel 15

### Kapseln mit 350 mg Wirkstoff

### Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

### Beispiel 16

### Suppositorien mit 100 mg Wirkstoff

| | |
|---|---|
| 1 Zäpfchen enthält: | |
| Wirkstoff | 100,0 mg |
| Polyethylenglykol (M.G. 1500) | 600,0 mg |
| Polyethylenglykol (M.G. 6000) | 460,0 mg |
| Polyethylensorbitanmonostearat | 840,0 mg |
| | 2 000,0 mg |

## Patentansprüche

1. Verbindungen der allgemeinen Formel 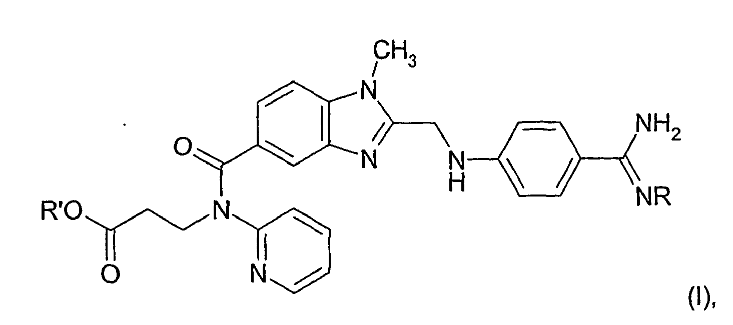 in der
a) R' ein Wasserstoffatom und R eine Methoxycarbonylgruppe
oder
b) R' ein Wasserstoffatom oder eine C₁₋₆-Alkylgruppe und R eine Hydroxygruppe, wobei die Alkylgruppen, die mehr als 2 Kohlenstoffatome enthalten, auch deren verzweigte Isomere einschließen,
bedeuten, deren Tautomere und deren Salze.

2. Verbindungen der allgemeinen Formel I gemäß Anspruch 1, in denen
a) R' ein Wasserstoffatom und R eine Methoxycarbonylgruppe
oder
b) R' ein Wasserstoffatom oder eine C₁₋₃-Alkylgruppe und R eine Hydroxygruppe, wobei die C₃-Alkylgruppe auch eine Isopropylgruppe mit einschließt,
bedeuten, deren Tautomere und deren Salze.

3. 1-Methyl-2-[4-(*N*-hydroxyamidino)-phenylaminomethyl]-benzimidazol-5-yl, carbonsäure-(*N*-2-pyridyl-*N*-2-ethoxycarbonylethyl)-amid, dessen Tautomere und dessen Salze.

4. 1-Methyl-2-[4-(*N*-methoxycarbonylamidino)-phenylaminomethyl]-benzimidazol-5-yl-carbonsäure-(*N*-2-pyridyl-*N*-2-hydroxycarbonylethyl)-amid, dessen Tautomere und dessen Salze.

5. Physiologisch verträgliche Salze der Verbindungen gemäß den Ansprüchen 1 bis 4.

6. Das Maleat, das Hydrochlorid und das Methansulfonat der Verbindung 1-Methyl-2-[4-(*N*-hydroxyamidino)-phenylaminomethyl]-benzimidazol-5-yl-carbonsäure-(*N*-2-pyridyl-*N*-2-ethoxycarbonylethyl)-amid.

7. Das Maleat, das Methansulfonat und das Natriumsalz der Verbindung 1-Methyl-2-[4-(*N*-methoxycarbonylamidino)-phenylaminomethyl]-benzimidazol-5-yl-carbonsäure-(*N*-2-pyridyl-*N*-2-hydroxycarbonylethyl)-amid.

8. Arzneimittel, enthaltend eine Verbindung nach mindestens einem der Ansprüche 1 bis 4 oder ein Salz gemäß einem der Ansprüche 5 bis 7 neben gegebenenfalls einem oder mehreren inerten Trägerstoffen und/oder Verdünnungsmitteln.

9. Arzneimittel gemäß Anspruch 8 zur subkutanen Anwendung.

10. Verwendung einer Verbindung nach mindestens einem der Ansprüche 1 bis 4 oder ein Salz gemäß einem der Ansprüche 5 bis 7 zur Herstellung eines Arzneimittels mit einer einer thrombinhemmender Wirkung.

11. Verwendung gemäß Anspruch 10 zur Herstellung eines Arzneimittels zur subkutanen Anwendung.

12. Verfahren zur Herstellung eines Arzneimittels gemäß Anspruch 8 oder 9, **dadurch gekennzeichnet, daß** auf nichtchemischem Wege eine Verbindung nach mindestens einem der Ansprüche 1 bis 4 oder ein Salz gemäß einem der Ansprüche 5 bis 7 in einen oder mehrere inerte Trägerstoffe und/oder Verdünnungsmittel eingearbeitet wird.

13. Verfahren zur Herstellung der Verbindungen gemäß den Ansprüchen 1 bis 7, **dadurch gekennzeichnet, daß**
a. zur Herstellung einer Verbindung der allgemeinen Formel I, in der R eine Hydroxygruppe darstellt,
eine gegebenenfalls im Reaktionsgemisch gebildete Verbindung der allgemeinen Formel 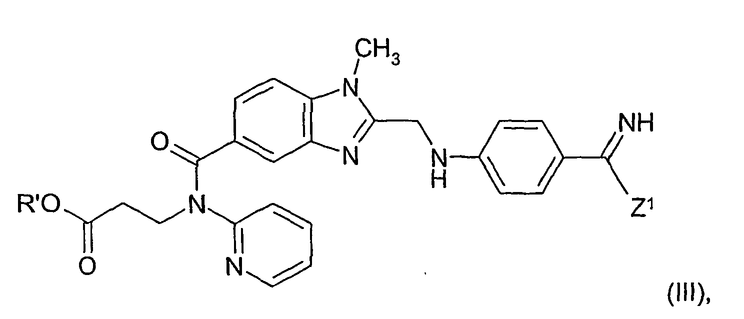 in der
R' gemäss der Ansprüchen 1 und 2 definiert ist und
Z₁ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt, mit einem Amin der allgemeinen Formel
H₂N - OH (IV)
umgesetzt wird, oder
b. zur Herstellung einer Verbindung der allgemeinen Formel I, in der R' Wasserstoff bedeutet,
eine Verbindung der allgemeinen Formel 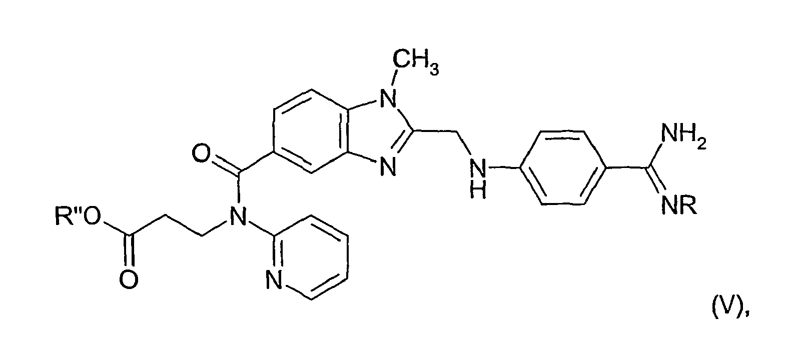 in der
R gemäss den Ansprüchen 1 und 2 definiert ist und
R" eine durch Hydrolyse, Behandeln mit einer Säure oder Base, Thermolyse oder Hydrogenolyse in eine Carboxylgruppe überführbare Gruppe bedeutet,
mittels Hydrolyse, Behandeln mit einer Säure oder Base, Thermolyse oder Hydrogenolyse in eine Verbindung der allgemeinen Formel I übergefürt wird, in der R' Wasserstoff bedeutet, oder
c. zur Herstellung einer Verbindung der allgemeinen Formel I, in der R eine Methoxycarbonylgruppe bedeutet,
eine Verbindung der allgemeinen Formel 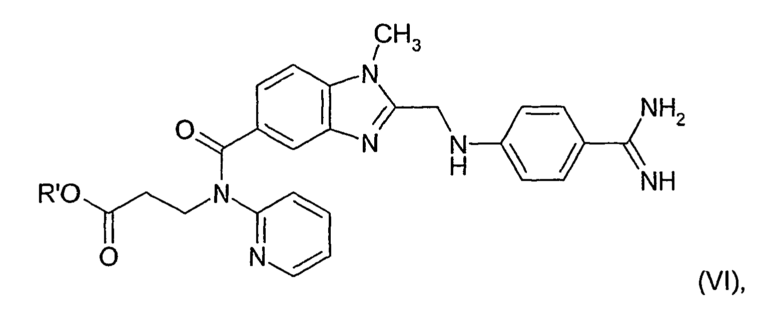 in der
R' gemäss den Ansprüchen 1 und 2 definiert ist, mit einer Verbindung der allgemeinen Formel
Z₂ - COOCH₃ , (VII)
in der
Z₂ eine nukleofuge Austrittsgruppe wie ein Halogenatom, z.B. ein Chlor-, Brom- oder Jodatom, bedeutet, umgesetzt wird,
und gewüschtenfalls die erhaltenen Verbindungen der Formel I in ihre Salze, insbesondere für die pharmazeutische Anwendung in ihre physiologisch verträglichen Salze mit anorganischen oder organischen Säuren oder Basen, übergeführt werden.

## Claims

1. Compounds of general formula 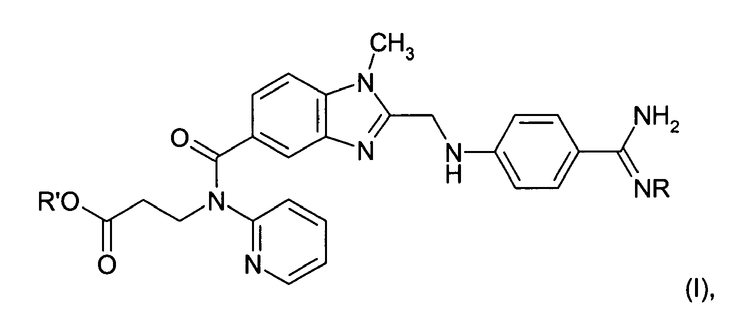 wherein
a) R' denotes a hydrogen atom and R denotes a methoxycarbonyl group
or
b) R' denotes a hydrogen atom or a C₁₋₆-alkyl group and R denotes a hydroxy group,
where the alkyl groups which contain more than 2 carbon atoms also include the branched isomers thereof,
the tautomers and the salts thereof.

2. Compounds of general formula I according to claim 1, wherein
a) R' denotes a hydrogen atom and R denotes a methoxycarbonyl group
or
b) R' denotes a hydrogen atom or a C₁₋₃-alkyl group and R denotes a hydroxy group, while the C₃-alkyl group also includes an isopropyl group,
the tautomers and the salts thereof.

3. 1-Methyl-2-[4-(*N*-hydroxyamidino)-phenylaminomethyl]-benzimidazol-5-yl-carboxylic acid-(*N*-2-pyridyl-*N*-2-ethoxycarbonylethyl)-amide, the tautomers and the salts thereof.

4. 1-Methyl-2-[4-(*N*-methoxycarbonylamidino)-phenylaminomethyl]-benzimidazol-5-yl-carboxylic acid-(*N*-2-pyridyl-*N*-2-hydroxycarbonylethyl)-amide, the tautomers and the salts thereof.

5. Physiologically acceptable salts of the compounds according to claims 1 to 4.

6. The maleate, the hydrochloride and the methanesulphonate of the compound 1-methyl-2-[4-(*N*-hydroxyamidino)-phenylaminomethyl]-benzimidazol-5-yl-carboxylic acid-(*N*-2-pyridyl-*N*-2-ethoxycarbonylethyl)-amide.

7. The maleate, the methanesulphonate and the sodium salt of the compound 1-methyl-2-[4-(*N*-methoxycarbonylamidino)-phenylaminomethyl]-benzimidazol-5-yl-carboxylic acid-(*N*-2-pyridyl-*N*-2-hydroxycarbonylethyl)-amide.

8. Pharmaceutical compositions containing a compound according to at least one of claims 1 to 4 or a salt according to one of claims 5 to 7, optionally together with one or more inert carriers and/or diluents.

9. Pharmaceutical compositions according to claim 8 for subcutaneous administration.

10. Use of a compound according to at least one of claims 1 to 4 or a salt according to one of claims 5 to 7 for preparing a pharmaceutical composition with a thrombin-inhibiting activity.

11. Use according to claim 10 for preparing a pharmaceutical composition for subcutaneous administration.

12. Process for preparing a pharmaceutical composition according to claim 8 or 9, **characterised in that** by a non-chemical method a compound according to at least one of claims 1 to 4 or a salt according to one of claims 5 to 7 is incorporated in one or more inert carriers and/or diluents.

13. Process for preparing the compounds according to claims 1 to 7, **characterised in that**
a. In order to prepare a compound of general formula I wherein R denotes a hydroxy group:
a compound of general formula 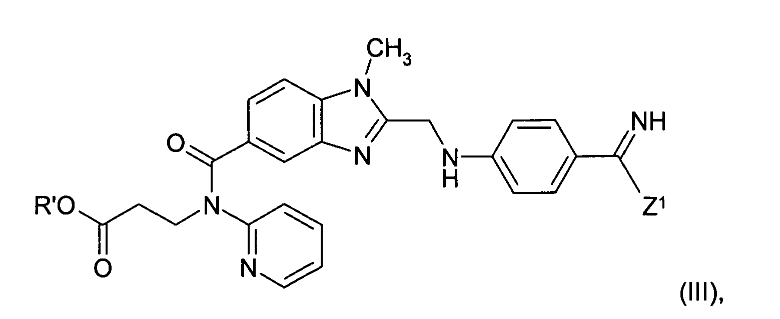 optionally formed in the reaction mixture,
wherein
R' is defined in accordance with claims 1 and 2 and
Z₁ denotes an alkoxy or aralkoxy group such as the methoxy, ethoxy, n-propoxy, isopropoxy or benzyloxy group or an alkylthio or aralkylthio group such as the methylthio, ethylthio, n-propylthio or benzylthio group,
is reacted with an amine of general formula
H₂N - OH (IV),
or
b. In order to prepare a compound of general formula I wherein R' denotes hydrogen:
a compound of general formula 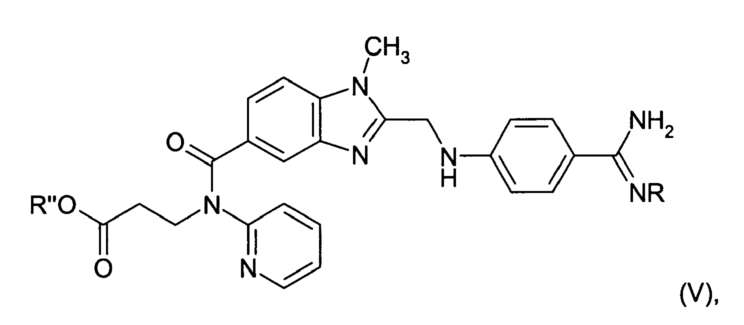
wherein
R is defined in accordance with claims 1 and 2 and
R" denotes a group which can be converted into a carboxyl group by hydrolysis, treatment with an acid or base, thermolysis or hydrogenolysis,
is converted into a compound of general formula I wherein R' denotes hydrogen, by hydrolysis, treatment with an acid or base, thermolysis or hydrogenolysis, or
c. In order to prepare a compound of general formula I wherein R denotes a methoxycarbonyl group:
a compound of general formula 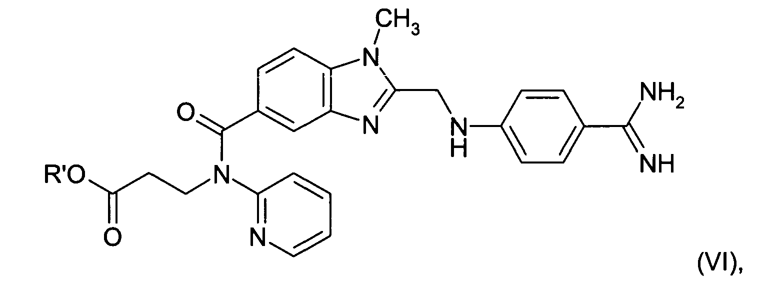
wherein
R' is defined in accordance with claims 1 and 2 and
is reacted with a compound of general formula
Z₂ - COOCH₃ (VII),
wherein
Z₂ denotes a nucleofugic leaving group such as a halogen atom, e.g. a chlorine, bromine or iodine atom,
and if desired the compounds of formula I obtained are converted into the salts thereof, particularly, for pharmaceutical use, into the physiologically acceptable salts thereof with inorganic or organic acids or bases.

## Revendications

1. Composés de formule générale 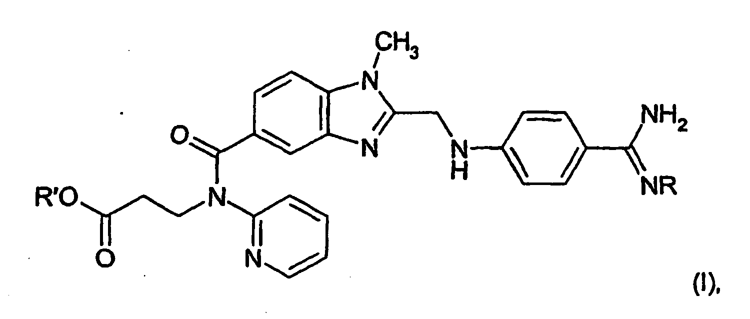 où
a) R' représente un atome d'hydrogène et R représente un groupe méthoxycarbonyle
ou
b) R' représente un atome d'hydrogène ou un groupe C₁₋₆-alkyle et R représente un groupe hydroxy, où les groupes alkyle, qui contiennent plus de 2 atomes de carbone, incluent aussi leurs isomères ramifiés, leurs tautomères et leurs sels.

2. Composés de formule générale I selon la revendication 1 où
a) R' représente un atome d'hydrogène et R représente un groupe méthoxycarbonyle
ou
b) R' représente un atome d'hydrogène ou un groupe C₁₋₃-alkyle et R représente un groupe hydroxy, où le groupe C₃-alkyle inclut aussi un groupe isopropyle, leurs tautomères et leurs sels.

3. (*N*-2-pyridyl-*N*-2-éthoxycarbonyléthyl)-amide d'acide 1-méthyl-2-[4-(*N*-hydroxyamidino)-phénylaminométhyl]-benzimidazol-5-yl-carboxylique, ses tautomères et ses sels.

4. (*N*-2-pyridyl-*N*-2-hydroxycarbonyléthyl)-amide d'acide 1-méthyl-2-[4-(*N*-méthoxycarbonylamidino)-phénylaminométhyl]-benzimidazol-5-yl-carboxylique, ses tautomères et ses sels.

5. Sels physiologiquement acceptables des composés selon les revendications 1 à 4.

6. Maléate, chlorhydrate et méthanesulfonate du composé (N-2-pyridyl-*N*-2-éthoxycarbonyléthyl)-amide d'acide 1-méthyl-2-[4-(*N*-hydroxy-amidino)-phénylaminométhyl]-benzimidazol-5-yl-carboxylique.

7. Maléate, méthanesulfonate et sel de sodium du composé (*N*-2-pyridyl-*N*-2-hydroxycarbonyléthyl)-amide d'acide 1-méthyl-2-[4-(*N*-méthoxy-carbonylamidino)-phénylaminométhyl]-benzimidazol-5-yl-carboxylique.

8. Médicament contenant un composé selon au moins l'une des revendications 1 à 4 ou sel selon l'une des revendications 5 à 7 outre éventuellement un ou plusieurs supports et/ou diluants inertes.

9. Médicament selon la revendication 8 pour l'utilisation sous-cutanée.

10. Utilisation d'un composé selon au moins l'une des revendications 1 à 4 ou d'un sel selon l'une des revendications 5 à 7 pour la production d'un médicament ayant un effet inhibant la thrombine.

11. Utilisation selon la revendication 10 pour la production d'un médicament pour l'utilisation sous-cutanée.

12. Procédé de production d'un médicament selon la revendication 8 ou 9 **caractérisé en ce que**, par voie non chimique, un composé selon au moins l'une des revendications 1 à 4 ou un sel selon l'une des revendications 5 à 7 est incorporé dans un ou plusieurs supports et/ou diluants inertes.

13. Procédé de production des composés selon les revendications 1 à 7
**caractérisé en ce que**
a. pour la production d'un composé de formule générale I où R représente un groupe hydroxy,
un composé éventuellement formé dans le mélange réactionnel de formule générale 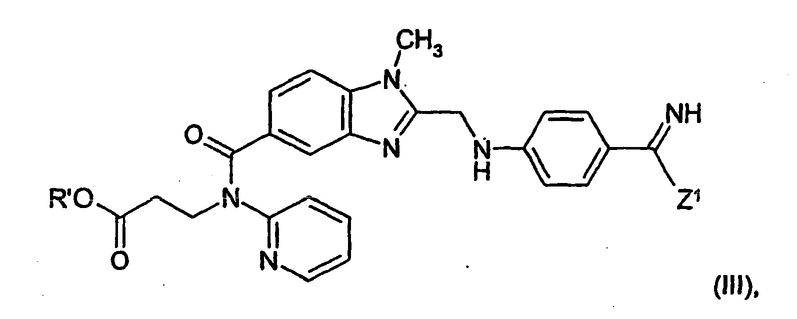 où
R' est défini selon les revendications 1 et 2 et
Z₁ représente un groupe alcoxy ou aralcoxy comme le groupe méthoxy, éthoxy, n-propoxy, isopropoxy ou benzyloxy ou un groupe alkylthio ou aralkylthio comme le groupe méthylthio, éthylthio, n-propylthio ou benzylthio, est mis à réagir avec une amine de formule générale
H₂N-OH (IV)
ou
b. pour la production d'un composé de formule générale I où R' représente l'hydrogène,
un composé de formule générale 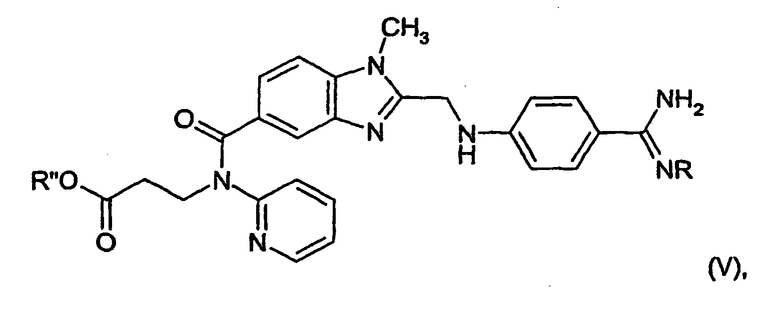 où
R est défini selon les revendications 1 et 2 et
R" représente un groupe convertible en un groupe carboxyle par hydrolyse, traitement avec un acide ou une base, thermolyse ou hydrogénolyse,
est converti par hydrolyse, traitement avec un acide ou une base, thermolyse ou hydrogénolyse en un composé de formule générale I où R' représente l'hydrogène, ou
c. pour la production d'un composé de formule générale I où R représente un groupe méthoxycarbonyle,
un composé de formule générale 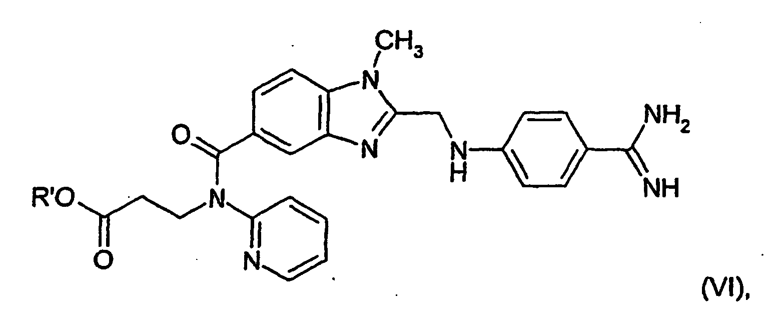 où
R' est défini selon les revendications 1 et 2, est mis à réagir avec un composé de formule générale
Z₂-COOCH₃ (VII)
où
Z₂ représente un groupe partant nucléofuge comme un atome d'halogène, par exemple un atome de chlore, de brome ou d'iode,
et si on le souhaite les composés de formule I obtenus sont convertis en leurs sels, en particulier pour l'utilisation pharmaceutique en leurs sels physiologiquement acceptables avec des acides ou des bases inorganiques ou organiques.
